(19) Europäisches Patentamt
European Patent Office
Office européen des brevets

(11) **EP 3 524 051 A1**

(12) **EUROPEAN PATENT APPLICATION**

(43) Date of publication:
**14.08.2019 Bulletin 2019/33**

(21) Application number: **18161269.8**

(22) Date of filing: **12.03.2018**

(51) Int Cl.:
*A01N 1/02* (2006.01)      *A23L 3/00* (2006.01)
*A01N 63/00* (2006.01)      *A01N 63/02* (2006.01)
*A61K 35/745* (2015.01)     *C12N 1/00* (2006.01)
*A23L 33/125* (2016.01)     *A01N 25/28* (2006.01)
*A01P 1/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.02.2018  US 201862629173 P**

(71) Applicant: **DuPont Nutrition Biosciences ApS
1411 Copenhagen K (DK)**

(72) Inventor: **SREENATH, Hassan
Madison, Wisconsin 53711 (US)**

(74) Representative: **DuPont EMEA
Langebrogade 1
1411 Copenhagen K (DK)**

(54) **MATRICIAL MICROENCAPSULATION COMPOSITIONS**

(57)      This specification relates to compositions and processes useful for stabilizing and preserving bacteria, particularly *Bifidobacteria*.

**EP 3 524 051 A1**

**Description**

FIELD

This specification relates to compositions and processes useful for stabilizing and preserving bacteria, particularly *Bifidobacteria.*

BACKGROUND

*Bifidobacteria* are gram-positive, non-acid-fast, non-spore-forming, non-motile, catalase negative rods of irregular shape. They are generally anaerobic chemoorganotrophs that metabolize a variety of carbohydrates through fermentation to produce organic acids but not gas (Scardovi, 1986). *Bifidobacteria* have been isolated from multiple sources, including food, sewage, insects, the human oral cavity and the guts of both humans and animals (Lamendella et al. 2008). Some species have been isolated from both human and animal guts, showing the ability to grow in different hosts. Other species have only been isolated from the gut of certain animal species (*e.g.*, rabbits, cows and chickens), demonstrating a highly specialized adaptation to specific ecological environments (Turrani et al 2009a; 2009b; Ventura et al. 2011). This highly evolved adaptation to growth in the gut is illustrated by the fact that strains can ferment complex carbohydrates for growth that are not digestible by the host (Schell, 2002; Locascio et al. 2010; Pokusaeva et al., 2011). Genetic analysis has revealed numerous oligosaccharide transporters and glycosyl hydro lases that can account for up to 10% of the genomic content of some strains (Ventura et al., 2007).

*Bifidobacterium animalis* subsp. *lactis* Bi-07 (also known as "*Bifidobacterium lactis* Bi-07" or "*B. lactis* Bi-07" or "Bi-07") is a particular *Bifidobacteria* strain which is publicly available, has been well characterised in the art and deposited at the American Type Culture Collection (ATCC) as Strain SD5220 (*see, e.g.,* Stahl and Barrangou 2009)).

It is well known that *Bifidobacteria* are useful inhabitants of the human intestine. In the intestine, these bacteria generally produce, for example, lactic acid and acetic acid, which lower the pH in the intestinal tract. Consequently, they tend to be beneficial for precluding local settlement of pathogenic organisms.

*Bifidobacteria* are often used as part of probiotic compositions, which aim to modulate the composition of a host's internal flora to improve the host's health through, for example, the enhancement of general host defences, as well as education and modulation of the immune system. Examples of probiotic compositions include foods, such as dairy products, and dietary supplements. Species of *Lactobacilli* and *Bifidobacteria* are the most widely used probiotic bacteria added to commercial bioactive products (Yang et al. 2008), with one of the most important commercial species being *B*. *lactis* (Oberg et al. 2011).

In addition, *Bifidobacteria* also have been reported to have beneficial effects in clinical backgrounds. For example, US9055763 discusses the use of *B. lactis* Bi-07 to treat gastrointestinal disorders.

Demand for products made using and/or comprising *Bifidobacteria* continues to increase worldwide. The stability of *Bifidobacteria,* in particular *B. lactis* Bi-07, as currently produced, tends to be inconsistent. Thus, there is a need for compositions comprising *Bifidobacteria* that, for example, exhibit greater stability following stressful processes necessary for long-term storage such as freezing and freeze-drying.

SUMMARY

Briefly, this specification discloses, in part, compositions for stabilizing probiotic bacteria, such as *Bifidobacteria.* The composition comprises a carbohydrate substrate, a potassium salt and a plant-based food oil. This specification also discloses, in part, processes for making and using such compositions.

In some embodiments, the composition for stabilizing probiotic bacteria also comprises one or more protein rich substrates.

In some embodiments, the composition for stabilizing probiotic bacteria also comprises one or more dietary fibre rich substrates.

In some embodiments, the composition for stabilizing probiotic bacteria also comprises a second carbohydrate substrate.

This specification also discloses, in part, a matricial microencapsulation ("MME") formulation comprising a probiotic bacterial culture and a composition for stabilizing probiotic bacteria as disclosed in this specification.

In some embodiments, at least 60% (by weight) of the components of the formulation are in liquid form.

This specification also discloses, in part, a process for preserving a probiotic bacterial culture, such as a *Bifidobacteria* culture. In some embodiments, the process comprises:

admixing a probiotic bacterial culture with a composition for stabilizing probiotic bacteria as disclosed in this specification to form an MME formulation as disclosed in this specification, and

subjecting the formulation to lyophilization.

In some embodiments, the probiotic bacterial culture is in the form of a frozen pellet before the admixing step. In some such embodiments, the frozen pellet is melted before the admixing step.

In some such embodiments, at least a portion of the drying cycle during lyophilization is conducted using a low pressure drying cycle having a pressure of from 50 to 150 mTorr and a temperature of from 2 to 32°C. In some embodiments, essentially all *(e.g.,* 90%) the drying cycle during lyophilization is conducted at a pressure of from 50 to 150 mTorr and a temperature of from 2 to 32°C. In some embodiments, all the drying cycle during lyophilization is conducted at a pressure of from 50 to 150 mTorr and a temperature of from 2 to 32°C.

In some embodiments, at least a portion of the drying during lyophilization is conducted using a high pressure drying cycle having a pressure of from 900 to 1100 mTorr and a temperature of from 22 to 28°C. In some embodiments, essentially all (*e.g.,* 90%) the drying during lyophilization is conducted at a pressure of from 900 to 1100 mTorr and a temperature of from 22 to 28°C. In some embodiments, all the drying during lyophilization is conducted at a pressure of from 900 to 1100 mTorr and a temperature of from 22 to 28°C.

This specification also discloses, in part, a lyophilized probiotic composition prepared by a process disclosed in this specification. In some embodiments, the lyophilized probiotic composition has at least 20% cell survival when stored for at least 30 days at a storage temperature of at least 38°C and/or at least 10% cell survival when stored for at least 60 days at a storage temperature of at least 38°C.

This specification also discloses, in part, a process for preparing a food product, dietary supplement or medicament. The process comprises admixing an excipient with probiotic bacteria prepared by a process described in this specification.

This specification also discloses, in part, a process for preparing a food product, dietary supplement or medicament. The process comprises admixing a prebiotic with probiotic bacteria prepared by a process described in this specification.

This specification also discloses, in part, a process for preparing a food product. The process comprises admixing a food ingredient with probiotic bacteria prepared by a process described in this specification.

In some of the above embodiments, the probiotic bacteria comprises *Bifidobacteria.*

In some of the above embodiments, the *Bifidobacteria* comprises *B. lactis.*

In some of the above embodiments, the *Bifidobacteria* comprises *B. lactis* Bi-07.

Further benefits of the teachings of this specification will be apparent to one skilled in the art from reading this specification.

BRIEF DESCRIPTION OF THE FIGURES

**Figure 1** shows the physical appearance of lyophilized pellets of *B. lactis* Bi-07 after a high pressure (~1000 mTorr) drying cycle (Left Hand Side) or a low pressure (~100 mTorr) drying cycle (Right Hand Side).

**Figure 2** shows an SEM image of lyophilised *B. lactis* Bi-07 when in a formulation with 30% sucrose & potassium phosphate control cryoprotectant (SKP cryoprotectant).

**Figure 3** shows an SEM image of lyophilised *B. lactis* Bi-07 when in a formulation comprising sucrose and a potassium buffer but without any plant-based food oils.

**Figure 4** shows an SEM image of lyophilised *B. lactis* Bi-07 when in a formulation comprising sucrose, a potassium buffer and canola oil.

DETAILED DESCRIPTION

This detailed description is intended to acquaint others skilled in the art with Applicant's invention, its principles, and its practical application so that others skilled in the art may adapt and apply Applicant's invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.

***Stabilising composition***

In a broad aspect, this specification discloses a composition for stabilizing probiotic bacteria comprising:

i) a carbohydrate substrate (also sometimes identified in this specification as "a first carbohydrate"),

ii) a potassium salt, and
iii) a plant-based food oil.

In some embodiments, the probiotic bacteria is *Bifidobacteria.* In some embodiments, the *Bifidobacteria* is *B. lactis.* In some embodiments, the *Bifidobacteria* is *B. lactis* Bi-07.

The term "carbohydrate" as used in this specification refers to a class of various, usually neutral compounds consisting of carbon, hydrogen and oxygen. The term "carbohydrate substrate" refers to any molecule or composition which provides a source of carbohydrates for a composition disclosed in this specification.

In some embodiments, the carbohydrate substrate comprises one or more disaccharide sugars.

In some embodiments, the carbohydrate substrate comprises trehalose.

In some embodiments, the carbohydrate substrate comprises sucrose.

In some embodiments, the carbohydrate substrate comprises lactose.

In some embodiments, the carbohydrate substrate comprises a sweet whey.

As used in this specification, "whey" refers to the liquid remaining after milk has been curdled and strained. It may be "sweet" or "acid", and contains mainly lactose in water, with minerals and protein.

The term "whey protein" refers specifically to the protein component of whey. It is typically a mixture of beta-lactoglobulin (~65%), alpha-lactalbumin (~25%), and serum albumin (~8%)

In some embodiments, the carbohydrate substrate comprises one or more oligosaccharides and/or polysaccharides. In some embodiments, the one or more oligosaccharide and/or polysaccharides is/are selected from maltodextrin, cello-dextrin and inulin.

In some embodiments, the carbohydrate substrate comprises a processed carbohydrate selected from high fructose corn syrup, grape juice extract, pine apple juice concentrate, cranberry pomace, apple pomace and beet sugar extract.

In some embodiments, the carbohydrate substrate comprises a fermented carbohydrate selected from corn steep liquor and molasses.

"Potassium buffer salt" as used in this specification, refers to an ionic compound comprising potassium and suitable for use in a buffer solution. In some embodiments, the potassium buffer salt is selected from monopotassium phosphate, dipotassium phosphate, tripotassium phosphate, mono potassium citrate, dipotassium citrate, potassium acetate, potassium formate, potassium aspartate, potassium lactate, potassium ascorbate and potassium caseinate. In some embodiments, the potassium buffer salt is dipotassium phosphate. In some embodiments, a mixture of two or more potassium buffer salts are used.

As used in this specification, the term "plant-based food oil" refers to an oil suitable for human consumption and derived from plants. Oils, as known in the art, are generally compositions made up of triacylglycerides. In some embodiments, the plant-based food oil can have a smoke point of at least 160°C. A "smoke point" is a temperature at which an oil begins to break down to glycerol and free fatty acids, and begins to lose flavor and nutritional value. Plant-based food oils that can be used in the compositions disclosed in this specification generally include, for example, canola oil, corn oil, mustard oil, olive oil, palm oil, palm kernel oil, peanut oil, safflower oil, sesame oil, soybean oil, almond oil, cottonseed oil, grape seed oil, sunflower oil, and mixtures thereof.

In some embodiments, the plant-based food oil is selected from flax oil, canola oil, soybean oil, coconut oil, olive oil, and sunflower oil.

In some embodiments, the plant-based food oil is canola oil.

In some embodiments, the plant-based food oil is flax oil.

As used in this specification, "flax oil", also known as linseed oil or flaxseed oil, refers to colorless to yellowish oil obtained from the dried ripe seeds of the flax plant (*Linum usitatissimum*).

As used in this specification, "sunflower oil" refers to non-volatile oil made from sunflower *(Helianthus annuus)* seeds, typically by compressing the seeds.

As used in this specification, "olive oil" refers to the oil obtained from olives (the fruit of *Olea europaea*). Typically, the oil produced by pressing whole olives.

As used in this specification, "soybean oil" refers to oil extracted from soybean seeds (*Glycine max*).

As used in this specification, "coconut oil" refers to oil extracted from the kernel or meat of matured coconuts harvested from a coconut palm (*Cocos nucifera*).

As used in this specification, "canola oil" refers to oil derived from seeds of a cultivar of either Rapeseed (*Brassica napus L.*) or field mustard (*Brassica campestris L.* or *Brassica Rapa var.*).

In some embodiments, the composition for stabilizing probiotic bacteria further comprises a protein rich substrate.

The term "protein rich substrate" refers to any molecule or composition which provides a source of protein and/or amino acids for a composition disclosed in this specification. In some embodiments, the protein rich substrate comprises at least 10% (by weight) proteins and/or amino acids. In some embodiments, the protein rich substrate comprises at least 20% (by weight) proteins and/or amino acids. In some embodiments, the protein rich substrate comprises at least 30% (by weight) proteins and/or amino acids. In some embodiments, the protein rich substrate comprises at least 40% (by weight) proteins and/or amino acids. In some embodiments, the protein rich substrate comprises at least 50% (by weight) proteins and/or amino acids. In some embodiments, the protein rich substrate comprises at least 60% (by weight) proteins and/or amino acids. In some embodiments, the protein rich substrate comprises at least 70% (by weight) proteins and/or amino acids. In some embodiments, the protein rich substrate comprises at least 80% (by weight) proteins and/or amino acids. In some embodiments, the protein rich substrate comprises at least 90% (by weight) proteins and/or amino acids. In some embodiments, the protein rich substrate comprises 100% proteins and/or amino acids.

Various suitable protein rich substrates are generally known in the art and commercially available.

In some embodiments, the protein rich substrate is selected from sweet whey, whey protein, Soybean protein, Cocoa, brown rice protein, Fenugreek seed powder, whole egg protein, egg white protein, Zein protein (for example, Zein protein derived from corn), pea protein and hemp seed powder.

In some embodiments, the protein rich substrate comprises hemp seed powder.

In some embodiments, the composition for stabilizing probiotic bacteria further comprises a dietary fiber rich substrate.

The term "dietary fiber rich substrate" refers to any molecule or composition which provides a source of dietary fiber for the composition disclosed in this specification. Dietary fibers are well known in the art and include compounds, usually derived from plants, which resist human digestive enzymes, such as lignin, some polysaccharides, inulin, some forms of resistant starch and inulin. In some embodiments, the dietary fiber rich substrate comprises at least 10% (by weight) dietary fiber compounds. In some embodiments, the dietary fiber rich substrate comprises at least 20% (by weight) dietary fiber compounds. In some embodiments, the dietary fiber rich substrate comprises at least 30% (by weight) dietary fiber compounds. In some embodiments, the dietary fiber rich substrate comprises at least 40% (by weight) dietary fiber compounds. In some embodiments, the dietary fiber rich substrate comprises at least 50% (by weight) dietary fiber compounds. In some embodiments, the dietary fiber rich substrate comprises at least 60% (by weight) dietary fiber compounds. In some embodiments, the dietary fiber rich substrate comprises at least 70% (by weight) dietary fiber compounds. In some embodiments, the dietary fiber rich substrate comprises at least 80% (by weight) dietary fiber compounds. In some embodiments, the dietary fiber rich substrate comprises at least 90% (by weight) dietary fiber compounds. In some embodiments, the dietary fiber rich substrate comprises at least 100% dietary fiber compounds.

Various suitable dietary fiber rich substrates are generally known in the art and commercially available.

In some embodiments, the dietary fiber rich substrate is selected from hemp seed powder, oat flour and ground flax seed.

In some embodiments, the dietary fiber rich substrate comprises hemp seed powder.

In some embodiments, the composition for stabilizing probiotic bacteria further comprises a second carbohydrate substrate (*i.e.*, a carbohydrate in addition to the carbohydrate discussed above (the "first carbohydrate")).

In some embodiments, the second carbohydrate substrate comprises a complex carbohydrate composition derived from one or more fruits.

In some embodiments, the second carbohydrate substrate is banana powder.

***Microencapsulation formulation***

In a broad aspect, this specification provides a matricial microencapsulation (MME) formulation comprising a probiotic bacterial culture and a composition for stabilizing probiotic bacteria as disclosed in this specification.

In some embodiments, the probiotic bacterial culture comprises a culture of *Bifidobacteria.* In some embodiments, the *Bifidobacteria* is *B. lactis.* In some embodiments, the *Bifidobacteria* is *B. lactis* Bi-07.

In some embodiments, the carbohydrate substrate is present in the MME formulation at a concentration of from 10% to 30% (by weight). In some embodiments, the carbohydrate is present in the formulation at a concentration of from 15% to 25% (by weight). In some embodiments, the carbohydrate is present in the formulation at a concentration of from 15% to 23% (by weight). In some embodiments, the carbohydrate is present in the formulation at a concentration of from 17% to 23% (by weight). In some embodiments, the carbohydrate is present in the formulation at a concentration of from 19% to 23% (by weight). In some embodiments, the carbohydrate is present in the formulation at a concentration of from 20% to 23% (by weight).

In some embodiments, the potassium buffer salt is present in the MME formulation at a concentration of 2% or less (by weight). In some embodiments, the potassium buffer salt is present in the formulation at a concentration of no greater than 1.5% (by weight). In some embodiments, the potassium buffer salt is present in the formulation at a concentration of no greater than 1% (by weight). In some embodiments, the potassium buffer salt is present in the formulation at a concentration of no greater than 0.75% (by weight).

In some embodiments, the plant-based food oil is present in the formulation at a concentration of up to 4.0% (by weight). In some embodiments, the plant-based food oil is present in the formulation is present at a concentration of from 0.5% to 4% (by weight). In some embodiments, the plant-based food oil is present in the formulation is present at a concentration of from 0.66% to 3.93% (by weight). In some embodiments, the plant-based food oil is present in the formulation at a concentration of from 0.66% to 3.0% (by weight). In some embodiments, the plant-based food oil is present in the formulation at a concentration of from 0.66% to 2.0% (by weight). In some embodiments, the plant-based food oil is present in the formulation at a concentration of from 1.0% to 2.0% (by weight). In some embodiments, the plant-based food oil is present in the formulation at a concentration of from 1.0% to 1.4% (by weight). In some embodiments, the plant-based food oil is present in the formulation at a concentration of from 1.3% to 1.4% (by weight).

In some embodiments, at least 60% (by weight) of the components in the MME formulation are in liquid form. In some embodiments, at least 70% (by weight) of the components in the formulation are in liquid form. In some embodiments, at least 75% (by weight) of the components in the formulation are in liquid form. In some embodiments, at least 80% (by weight) of the components are in liquid form.

In some embodiments, the pH of the MME formulation is from about 5 to about 7. In some embodiments, the pH of the formulation is from about 6 to about 6.5.

As the use of probiotic bacteria as a food supplement has become more widespread, there is an increasing need to be able to maintain the viability of the bacteria during storage, especially at room temperature, such that the convenience for the consumer and/or manufacturer is improved. Freezing and lyophilization (freeze-drying) are common techniques employed to maintain long term stability of probiotic bacteria. Both the processes, however, involve subjecting the bacteria to harsh temperatures and pressures, and, thus, can have adverse effects on at least the bacterial membrane integrity and protein structures, resulting in an overall decrease in viability. The microencapsulation technologies discussed in this specification aim to protect the bacteria during exposure to such conditions and during dehydration.

In some embodiments, the MME formulation is prepared by a process comprising blending a probiotic bacterial culture with a composition for stabilizing probiotic bacteria as disclosed in this specification.

In some embodiments, the MME formulations is capable of improving the viability and stability of the probiotic bacterial culture when the formulation is subjected to lyophilization.

In some embodiments, the MME formulations is capable of encapsulating the probiotic bacterial culture when subjected to lyophilization.

As used in this specification, "stable" and its derivative terms (*e.g.,* stability, stabilize, etc.) refers to relative stability under storage (*e.g.,* at 37°C or 38°C) after being subjected to lyophilization (also known as freeze-drying).

In some embodiments, the probiotic bacterial culture is not stable before being part of the MME formulation.

In some embodiments, the probiotic bacterial culture, after lyophilisation, can be characterized as exhibiting a cell survival of at least 20% when stored for 30 days at a temperature of 38°C and/or a cell survival of at least 10% when stored for 60 days at a temperature of 38°C.

### *Process for preserving*

In a broad aspect, this specification provides a process for preserving a probiotic bacterial culture, wherein the process comprises:

   admixing the probiotic bacterial culture with a composition for stabilizing probiotic bacteria as disclosed in this specification to form an MME formulation as disclosed in this specification, and
   subjecting the MME formulation to lyophilisation to form a lyophilized probiotic composition.

In some embodiments, the probiotic bacterial culture is a culture of *Bifidobacteria.* In some embodiments, the *Bifidobacteria* is *B. lactis.* In some embodiments, the *Bifidobacteria* is *B. lactis* Bi-07.

"Preserving" as used in this specification refers to increasing the stability (*e.g.*, during storage) after a preservation process such as lyophilization.

In some embodiments, the admixing of the probiotic bacterial culture with a composition for stabilizing probiotic bacteria is carried out using a blending process. Various suitable blenders are known in the art.

In some embodiments, the blending occurs over a duration of from about 60 sec to about 300 sec.

In some embodiments, the blending occurs over a duration of at least 90 sec.

In some embodiments, the blending incorporates a cooling period between mixing.

In some embodiments, the temperature during and after blending is maintained at from about 20°C to about 38°C. In some embodiments, the temperature during and after blending is maintained at from about 24°C to about 30°C.

"Lyophilization" (also known as freeze-drying or cryodessication) refers to a preservation technique generally well known in the art. In brief, following pre-treatment of a substance to form a formulation to be lyophilized, the formulation is generally first frozen, before being subjected to a vacuum. Under the vacuum, heat is typically applied such that the moisture in the formulation (in ice form) sublimes from the formulation. A secondary drying step may exist to remove any unfrozen water molecules, where the temperature is generally increased.

In some embodiments, at least a portion of the drying during the lyophilization is carried out at a pressure of from about 50 to about 150 mTorr and a temperature of from 2 to 32°C (low pressure drying cycle). In some embodiments, at least a portion of the drying during the lyophilization is conducted at a pressure of from 75 to 125 mTorr and a temperature of from 2 to 32°C. In some embodiments, at least a portion of the drying during the lyophilization is conducted at a pressure of about 100 mTorr and a temperature of from 2 to 32°C. In some embodiments, essentially all (*e.g.,* 90%) the drying during the lyophilization is carried out at a pressure of from about 50 to about 150 mTorr and a temperature of from 2 to 32°C (low pressure drying cycle). In some embodiments, essentially all (*e.g.,* 90%) the drying during the lyophilization is conducted at a pressure of from 75 to 125 mTorr and a temperature of from 2 to 32°C. In some embodiments, essentially all (*e.g.,* 90%) the drying during the lyophilization is conducted at a pressure of about 100 mTorr and a temperature of from 2 to 32°C. In some embodiments, all the drying during the lyophilization is carried out at a pressure of from about 50 to about 150 mTorr and a temperature of from 2 to 32°C (low pressure drying cycle). In some embodiments, all the drying during the lyophilization is conducted at a pressure of from 75 to 125 mTorr and a temperature of from 2 to 32°C. In some embodiments, all the drying during the lyophilization is conducted at a pressure of about 100 mTorr and a temperature of from 2 to 32°C.

In some embodiments, at least a portion of the drying cycle of the lyophilization is carried out at a pressure of from about 900 to about 1100 mTorr and a temperature of from 22°C to 28°C (high pressure drying cycle). In some embodiments, at least a portion of the drying cycle of the lyophilization is conducted at a pressure of from 950 to 1050 mTorr and a temperature of from 22 to 28°C. In some embodiments, at least a portion of the drying cycle of the lyophilization is conducted at a pressure of from about 1000 mTorr and temperature of from 22 to 28°C. In some embodiments, essentially all (*e.g.,* 90%) the drying cycle of the lyophilization is carried out at a pressure of from about 900 to about 1100 mTorr and a temperature of from 22°C to 28°C. In some embodiments, essentially all (*e.g.,* 90%) the drying cycle of the lyophilization is conducted at a pressure of from 950 to 1050 mTorr and a temperature of from 22 to 28°C. In some embodiments, essentially all (*e.g.,* 90%) the drying cycle of the lyophilization is conducted at a pressure of from about 1000 mTorr and temperature of from 22 to 28°C. In some embodiments, all the drying cycle of the lyophilization is carried out at a pressure of from about 900 to about 1100 mTorr and a temperature of from 22°C to 28°C. In some embodiments, all the drying cycle of the lyophilization is conducted at a pressure of from 950 to 1050 mTorr and a temperature of from 22 to 28°C. In some embodiments, all the drying cycle of the lyophilization is conducted at a pressure of from about 1000 mTorr and temperature of from 22 to 28°C.

In some embodiments, the probiotic bacterial culture is in the form of a frozen pellet before the admixing step. In some such embodiments, the frozen pellet is melted before the admixing step.

In some embodiments, the probiotic bacterial culture is in the form of a liquid culture before the admixing step.

In some embodiments, the probiotic bacterial culture has a bacterial content of at least $1 \times 10^{11}$ CFU/g.

This specification also discloses a lyophilized probiotic composition produced by the above process.

In some embodiments, the probiotic is *Bifidobacteria.* In some embodiments, the *Bifidobacteria* is *B. lactis.* In some embodiments, the *Bifidobacteria* is *B. lactis* Bi-07.

In some embodiments, the lyophilized probiotic compositions have a water activity ($a_w$) of from about 0.010 to about 0.310.

In some embodiments, the lyophilized probiotic compositions have a percent recovery of at least 30%. In some embodiments, the lyophilized probiotic compositions have a percent recovery of at least 40%. In some embodiments, the lyophilized probiotic compositions have a percent recovery of at least 50%. In some embodiments, the lyophilized probiotic compositions have a percent recovery of at least 60%. In some embodiments, the lyophilized probiotic compositions have a % recovery of at least 70%.

In some embodiments, the lyophilized probiotic compositions can be characterized as having a cell survival of at least 20% when stored for 30 days at a temperature of 38°C. In some embodiments, the lyophilized probiotic compositions can be characterized as having a cell survival of at least 30% when stored for 30 days at a temperature of 38°C. In some embodiments, the lyophilized probiotic compositions can be characterized as having a cell survival of at least 40% when stored for 30 days at a temperature of 38°C. In some embodiments, the lyophilized probiotic compositions can be characterized as having a cell survival of at least 50% when stored for 30 days at a temperature of 38°C.

In some embodiments the lyophilized probiotic compositions can be characterized as having a cell survival of at least 10% when stored for 60 days at a temperature of 38°C. In some embodiments, the lyophilized probiotic compositions can be characterized as having a cell survival of at least 20% when stored for 60 days at a temperature of 38°C. In some embodiments, the lyophilized probiotic compositions can be characterized as having a cell survival of at least 30% when stored for 60 days at a temperature of 38°C. In some embodiments, the lyophilized probiotic compositions can be characterized as having a cell survival of at least 40% when stored for 60 days at a temperature of 38°C.

In some embodiments, the lyophilized probiotic is further packaged to facilitate storage. In some embodiments, the packaging comprises aluminum foil.

In some embodiments, the lyophilized probiotic composition, particularly one comprising *Bifidobacteria,* made in accordance with a process disclosed in this specification is used as part of (or to make) a food, dietary supplement or medicament. In some embodiments, the composition is a food product. In some embodiments, the composition is a dietary supplement. In some embodiments, the composition is a medicament (or pharmaceutical composition). Such a composition may be in the form of a liquid or solid. In the latter instance, the product may, for example, be powdered and formed into tablets, granules or capsules or simply mixed with other ingredients.

A food, dietary supplement or medicament comprising *Bifidobacteria* prepared using a process disclosed in this specification typically comprises other ingredients besides the *Bifidobacteria.* For example, with respect to foods, the *Bifidobacteria* prepared using a process disclosed in this specification may be mixed with one or more food ingredients. And, with respect to medicaments, the *Bifidobacteria* prepared using a process disclosed in this specification may be mixed with one or more pharmaceutically acceptable excipients. In some embodiments, the *Bifidobacteria* is *B. lactis.* In some embodiments, the *Bifidobacteria* is *B. lactis* Bi-07.

Illustrative contemplated food compositions include, for example, any ingestible material selected from of milk, curd, milk based fermented products, acidified milk, yogurt, frozen yogurt, milk powder, milk based powders, milk concentrate, cheese, cheese spreads, dressings, beverages, ice-creams, fermented cereal based products, infant formulae, tablets, liquid bacterial suspensions, dried oral supplement, wet oral supplement, dry tube feeding and wet tube feeding that is produced by admixing *Bifidobacteria* with a specified food ingredient(s).

As used in this specification, the term "pharmaceutically acceptable excipient" may be, for example, a solid or liquid filler diluent or encapsulating substance that is suitable for administration to a human or animal and which is/are compatible with the probiotically active organism being used. The term "compatible" relates to components of the composition which are capable of being commingled with the *Bifidobacteria* in a manner enabling no interaction that would substantially reduce the probiotic efficacy of the organisms selected under ordinary use conditions. A pharmaceutically acceptable carrier must generally be of a sufficiently high purity and a low toxicity to render it suitable for administration to the human or animal being treated.

In some embodiments, a solid composition as described in this specification is a tablet, capsule or granulate (comprising a number of granules). The solid composition may be an oral dosage form. The tablets may be prepared by processes known in the art, and can be compressed, enterically coated, sugar coated, film coated or multiply compressed, and containing one or more suitable binders, lubricants, diluents, disintegrating agents, coloring agents, flouring agents, flow-inducing agents and melting agents. Capsules can generally be soft or hard capsules, have liquid and/or solid contents, and be prepared according to conventional techniques generally well known in the pharmaceutical industry. As one example, *Bifidobacteria* may be filled into a variety of capsules using a suitable filling machine. A solid composition as described in this specification may also be, for example, a pellet.

In some embodiments, the composition prepared with *Bifidobacteria* made by a process disclosed in this specification is a probiotic composition.

A contemplated daily dose for humans and animals of *Bifidobacterium* (particularly, *B. lactis*, including *B. lactis* Bi-07) prepared by a process described in this specification is from about $10^3$ to about $10^{14}$ CFU per day. In some embodiments, the daily dose is from $10^6$ to $10^{13}$ CFU per day. In some embodiments, the daily dose is from $10^8$ to $10^{12}$ CFU per day. In some embodiments, the daily dose is from $10^9$ to $10^{11}$ CFU per day.

In some embodiments, the food product, dietary supplement or medicament further comprises one or more prebiotic substances. Examples of contemplated prebiotic substances include fructo-oligosaccharides (FOS), inulin, galacto-oligosaccharides (GOS) and mannan-oligosaccharides (MOS).

### *Uses of MME formulations*

Also provided by this specification are uses of the MME formulations described above.

In general, the MME formulation is capable of providing stabilization of a probiotic bacterial culture such that the probiotic bacterial culture, following lyophilization, can be characterized as having an increased cell survival when stored for 30 days and/or 60 days at a temperature of 38°C compared to an otherwise identical formulation without a plant-based food oil at a temperature of 38°C. In some embodiments, the MME formulation is capable of providing stabilization of a probiotic bacterial culture such that the probiotic bacterial culture, following lyophilization, can be characterized as having a cell survival of at least 20% when stored for 30 days at a storage temperature of 38°C and/or a cell survival of at least 10% when stored for 60 days at a storage temperature of 38°C.

### EXAMPLES

The following examples are merely illustrative, and not limiting to this specification in any way.

***Cultures and Processes***

*Probiotic bacterial cultures*

Cultures of *B. lactis* Bi-07 were produced by fermentation techniques generally known in the art. All fermentations in these examples used the growth media shown in Table 1. The fermentation was conducted for from 12 to 16 hours and carried out at a temperature of 38°C and pH of about 6.0. The resulting culture was chilled to below 15°C before being concentrated (1:16). Concentration was performed by batch centrifugation using a Beckman Avanti J-26 XP centrifuge, JLA 8.1000 head at 6,000 rpm (RCF 12,227 x g) for 30 min followed by re-suspension in fermentate media supernatant to form a liquid cell concentrate.

**Table 1**

| Fermentation Medium | |
|---|---|
| **Ingredient** | **gram** |
| Dextrose and Corn Syrup Solids | 33 |
| Yeast Extract | 20 |
| Magnesium Sulphate | 7 |
| Ascorbic Acid | 1 |
| Vitamin B12 | 0.1 |
| Manganese Sulphate | 0.1 |
| **Ingredient** | **gram** |
| Cysteine | 0.5 |
| Polysorbate 80 | 5 |
| Water | to 1 L |

Both fresh liquid cell concentrates and thawed, frozen pellet cell concentrates of *B. lactis* Bi-07 were used in the below Examples for testing stability when mixed with a stabilizing composition described in this specification.

The fresh liquid cell concentrates were the liquid cell concentrates as described above, which were not frozen before being admixed with a stabilizing composition described in this specification and lyophilized.

With respect to the thawed frozen pellet cell concentrates, liquid cell concentrates as described above were first frozen in liquid nitrogen and stored at -80°C before being thawed (or "melted") and then admixed with a stabilizing composition described in this specification and lyophilized. The thawing was carried out by transferring the pellets to an aseptic steel vessel and thawing them at 25°C in a temperature-controlled water bath for approximately 30 min using a standard homogenizer fixed with a standard stirrer (IKA RW20 digital overhead stirrer) at speed of ~600 rpm.

The SKP control formulation used in these experiments consisted of ~70% (by weight) of the liquid cell concentrates as described above and ~30% (by weight) of the SKP cryoprotectant shown in **Table 2.** The SKP stabilized control formulation was lyophilized by freezing in liquid nitrogen, and then low pressure drying at a pressure of from about 50 to about 150 mTorr and a temperature of from 2 to 32°C under vacuum (*i.e.*, low pressure drying cycle conditions commonly used by conventional drying cycle processes).

**Table 2 - SKP cryoprotectant composition**

| Ingredient | Weight (g) |
|---|---|
| Water (at 65°C) | 251.2 |
| Sucrose | 240.0 |
| Mono-potassium phosphate | 47.72 |
| Di-potassium phosphate | 61.08 |
| **Total** | 600.0 |

*Lyophilization stability testing*

Encapsulating compositions were added to the unstabilised thawed probiotic bacteria or fresh liquid cultures and blended to form microencapsulation formulations described in this specification. The blended mix was frozen in liquid nitrogen and the resulting frozen pellets weighed and stored at -80°C until freeze-drying by low or high pressure cycles.

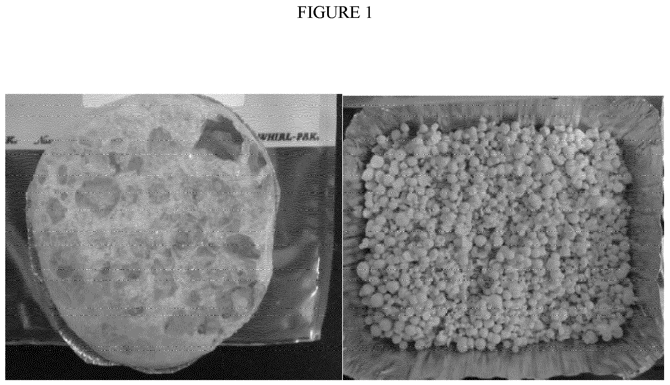

The frozen pellets were freeze-dried using a conventional freeze-drier with low pressure drying conditions (*e.g.*, a pressure of from 50 to 150 mTorr and a temperature of from 2 to 32°C) or high pressure drying conditions (*e.g.*, a pressure of from 900 to 1100 mTorr and a temperature of from 22 to 28°C) under vacuum. The two different drying conditions lead to different physical characteristics of the resulting pellet, as can be seen in **Figure 1.** The left-hand side of **Figure 1** shows that formulations undergoing high pressure drying cycle gave fused, collapsed, cakey pellets of probiotic bacteria (Plate 1A) usually with high water activity. However, the low pressure drying cycle gave free flowing probiotic bacterial pellets (right hand side of **Figure 1**) with low water activity.

The water activity ($a_w$) of the dried material (low pressure drying cycle or high pressure drying cycle) were measured on a Rotronic Hygrolab 3 meter at room temperature. A portion of the lyophilized material was enumerated immediately after freeze-drying (TO). Other portions of the lyophilized material were sealed in aluminum foil sachets and enumerated after incubation at 38°C for 14 days, 30 days and 60 days.

*Enumeration of B. lactis Bi-07*

Lyo cell counts in CFU (colony forming units) for the purposes of calculating % cell survival and Parker stability were obtained by pour plating with MRS Agar supplemented with 0.01% cysteine-HCl and incubated at 38°C under anaerobic conditions for 48-72 hours. Freeze-dried pellets with and without MME (stability material) were rehydrated in MRS broth for 30 min at room temperature (22-25°C) before subsequent dilutions and pour plating with MRS Agar supplemented with 0.01 % cysteine-HCl.

Percentage cell survival was calculated as follows:

$$\% \, Survival = \frac{Cell \; count \; (lyophilised) \; at \; 14 \; or \; 30 \; or \; 60 \; days \; (CFU/g)}{Cell \; count \; (lyophilised) \; at \; 0 \; days \; (CFU/g)} \times 100 \, \%$$

The Parker test for stability is a standard enumeration to assess stability. For this test, the freeze-dried sample was transferred to a foil sachet and sealed. The sample was incubated at 37-38°C for 14 days. Samples were then enumerated using the appropriate freeze-dried enumeration protocol. The following calculation was used to determine the Parker half-life:

$$Parker \; half\text{-}life \; (days) = 14/ \, (3.32 * (Log \; (T0 \; CFU/g) - Log \; (Parker \; (days) \; CFU/g)))$$

**Example 1 - Formulations**

The present disclosure contemplates a number of matricial microencapsulation (MME) formulations comprising a probiotic bacterial culture. Various such formulations have been observed to protect and stabilise probiotic bacteria through processes such as freeze-drying (lyophilisation). These formulations are listed below (with the percentages being weight percentages). In each case, the encapsulating composition was added to the unstabilised thawed probiotic bacteria or fresh liquid cultures (as described above) and then blended using a standard blender initially for from 10 to 200 sec at a temperature of from 20 to 38°C and a pH of from 6 to 6.5, allowed to cool for from 60 to 300 s, and blended again for from 10 to 60 sec at a temperature of from 24 to 30°C and a pH of from 6.0 to 6.5. The pH was adjusted and the temperature measured in each case during the blending to maintain the desired range. Formulations 1 to 3 are formulations often used for comparative purposes in MME studies.

| Formulation 1 | | |
|---|---|---|
| MME ingredient | % solids | % liquid |
| Thawed probiotic bacteria | 0 | 75 |
| Sucrose | 25 | 0 |

Formulation 2

| MME ingredient | % solids | % liquid |
|---|---|---|
| Thawed probiotic bacteria | 0 | 75 |
| Sucrose | 24.04 | 0 |
| Dipotassium phosphate | 0.96 | 0 |

Formulation 3

| MME ingredient | % solids | % liquid |
|---|---|---|
| Thawed probiotic bacteria | 0 | 75 |
| Trehalose | 24.04 | 0 |
| Dipotassium phosphate | 0.96 | 0 |

Formulation 4

| MME ingredient | % solids | % liquid |
|---|---|---|
| Thawed probiotic bacteria | 0 | 75 |
| Sucrose | 22.77 | 0 |
| Dipotassium phosphate | 0.91 | 0 |
| Canola oil | 0 | 1.32 |

Formulation 5

| MME ingredient | % solids | % liquid |
|---|---|---|
| Thawed probiotic bacteria | 0 | 75 |
| Sucrose | 22.77 | 0 |
| Dipotassium phosphate | 0.91 | 0 |
| Flax oil | 0 | 1.32 |

Formulation 6

| MME ingredient | % solids | % liquid |
|---|---|---|
| Thawed probiotic bacteria | 0 | 75 |
| Sucrose | 22.77 | 0 |
| Dipotassium phosphate | 0.91 | 0 |
| Olive oil | 0 | 1.32 |

Formulation 7

| MME ingredient | % solids | % liquid |
|---|---|---|
| Thawed probiotic bacteria | 0 | 75 |
| Sucrose | 22.77 | 0 |
| Dipotassium phosphate | 0.91 | 0 |
| Soybean oil | 0 | 1.32 |

Formulation 8

| MME ingredient | % solids | % liquid |
| --- | --- | --- |
| Thawed probiotic bacteria | 0 | 75 |
| Sucrose | 22.77 | 0 |
| Dipotassium phosphate | 0.91 | 0 |
| Coconut oil | 0 | 1.32 |

Formulation 9

| MME ingredient | % solids | % liquid |
| --- | --- | --- |
| Thawed probiotic bacteria | 0 | 75 |
| Trehalose | 22.77 | 0 |
| Dipotassium phosphate | 0.91 | 0 |
| Canola oil | 0 | 1.32 |

Formulation 10

| MME ingredient | % solids | % liquid |
| --- | --- | --- |
| Thawed probiotic bacteria | 0 | 75 |
| Trehalose | 22.77 | 0 |
| Dipotassium phosphate | 0.91 | 0 |
| Flax oil | 0 | 1.32 |

Formulation 11

| MME ingredient | % solids | % liquid |
| --- | --- | --- |
| Thawed probiotic bacteria | 0 | 75 |
| Sucrose | 22.77 | 0 |
| Dipotassium phosphate | 0.91 | 0 |
| Canola oil | 0 | 1.32 |

Formulation 12

| MME ingredient | % solids | % liquid |
| --- | --- | --- |
| Thawed probiotic bacteria | 0 | 75 |
| Sucrose | 23.41 | 0 |
| Dipotassium phosphate | 0.93 | 0 |
| Canola, flax or soybean oil | 0 | 0.66 |

Formulation 13

| MME ingredient | % solids | % liquid |
| --- | --- | --- |
| Thawed probiotic bacteria | 0 | 75 |
| Sucrose | 22.77 | 0 |
| Dipotassium phosphate | 0.91 | 0 |
| Canola, flax or soybean oil | 0 | 1.32 |

Formulation 14

| MME ingredient | % solids | % liquid |
| --- | --- | --- |
| Thawed probiotic bacteria | 0 | 75 |
| Sucrose | 22.17 | 0 |
| Dipotassium phosphate | 0.88 | 0 |
| Canola, flax or soybean oil | 0 | 1.95 |

Formulation 15

| MME ingredient | % solids | % liquid |
| --- | --- | --- |
| Thawed probiotic bacteria | 0 | 75 |
| Sucrose | 21.52 | 0 |
| Dipotassium phosphate | 0.86 | 0 |
| Canola, flax or soybean oil | 0 | 2.62 |

Formulation 16

| MME ingredient | % solids | % liquid |
| --- | --- | --- |
| Thawed probiotic bacteria | 0 | 75 |
| Sucrose | 20.26 | 0 |
| D potassium phosphate | 0.81 | 0 |
| Canola, flax or soybean oil | 0 | 3.93 |

Formulation 17

| MME ingredient | % solids | % liquid |
| --- | --- | --- |
| Thawed probiotic bacteria | 0 | 75.04 |
| Sucrose | 17.38 | 0 |
| Dipotassium phosphate | 0.70 | 0 |
| Brown rice protein | 4.92 | 0 |
| Canola oil | 0 | 1.96 |

Formulation 18

| MME ingredient | % solids | % liquid |
| --- | --- | --- |
| Thawed probiotic bacteria | 0 | 75.04 |
| Sucrose | 17.38 | 0 |
| Dipotassium phosphate | 0.70 | 0 |
| Fenugreek seed powder | 4.92 | 0 |
| Canola oil | 0 | 1.96 |

Formulation 19

| MME ingredient | % solids | % liquid |
| --- | --- | --- |
| Thawed probiotic bacteria | 0 | 75.02 |
| Sucrose | 17.23 | 0 |
| Dipotassium phosphate | 0.69 | 0 |

(continued)

| MME ingredient | % solids | % liquid |
|---|---|---|
| Soybean protein | 5.03 | 0 |
| Ground Flax | 0.69 | 0 |
| Canola oil | 0 | 1.34 |

Formulation 20

| MME ingredient | % solids | % liquid |
|---|---|---|
| Thawed probiotic bacteria | 0 | 75.02 |
| Sucrose | 17.23 | 0 |
| Dipotassium phosphate | 0.69 | 0 |
| Cocoa | 5.03 | 0 |
| Ground Flax seed | 0.69 | 0 |
| Canola oil | 0 | 1.34 |

Formulation 21

| MME ingredient | % solids | % liquid |
|---|---|---|
| Thawed probiotic bacteria | 0 | 75.02 |
| Sucrose | 17.23 | 0 |
| Dipotassium phosphate | 0.69 | 0 |
| Zein protein | 5.03 | 0 |
| Ground Flax seed | 0.69 | 0 |
| Canola oil | 0 | 1.34 |

Formulation 22

| MME ingredient | % solids | % liquid |
|---|---|---|
| Thawed probiotic bacteria | 0 | 75.02 |
| Sucrose | 17.23 | 0 |
| Dipotassium phosphate | 0.69 | 0 |
| Brown rice protein | 5.03 | 0 |
| Ground Flax seed | 0.69 | 0 |
| Canola oil | 0 | 1.34 |

Formulation 23

| MME ingredient | % solids | % liquid |
|---|---|---|
| Thawed probiotic bacteria | 0 | 75.02 |
| Sucrose | 17.23 | 0 |
| Dipotassium phosphate | 0.69 | 0 |
| Whole egg protein | 5.03 | 0 |
| Ground Flax seed | 0.69 | 0 |
| Canola oil | 0 | 1.34 |

Formulation 24

| MME ingredient | % solids | % liquid |
| --- | --- | --- |
| Thawed probiotic bacteria | 0 | 75.02 |
| Sucrose | 17.23 | 0 |
| Dipotassium phosphate | 0.69 | 0 |
| Egg white protein | 5.03 | 0 |
| Ground Flax seed | 0.69 | 0 |
| Canola oil | 0 | 1.34 |

Formulation 25

| MME ingredient | % solids | % liquid |
| --- | --- | --- |
| Thawed probiotic bacteria | 0 | 75.02 |
| Sucrose | 17.23 | 0 |
| Dipotassium phosphate | 0.69 | 0 |
| Ground Fenugreek seed | 5.03 | 0 |
| Ground Flax seed | 0.69 | 0 |
| Canola oil | 0 | 1.34 |

Formulation 26

| MME ingredient | % solids | % liquid |
| --- | --- | --- |
| Thawed probiotic bacteria | 0 | 75.02 |
| Sucrose | 17.23 | 0 |
| Dipotassium phosphate | 0.69 | 0 |
| Hemp seed powder | 5.03 | 0 |
| Ground Flax seed | 0.69 | 0 |
| Canola oil | 0 | 1.34 |

Formulation 27

| MME ingredient | % solids | % liquid |
| --- | --- | --- |
| Thawed probiotic bacteria | 0 | 90.1 |
| Hemp seed powder | 6.7 | 0 |
| Ground Flax seed | 0.9 | 0 |
| Di potassium phosphate | 0.7 | 0 |
| Flax oil | 0 | 1.6 |

Formulation 28

| MME ingredient | % solids | % liquid |
| --- | --- | --- |
| Thawed probiotic bacteria | 0 | 80.0 |
| Sweet Whey | 16.94 | 0 |
| Di- potassium phosphate | 1.75 | 0 |
| Flax oil | 0 | 1.31 |

Formulation 29

| MME ingredient | % solids | % liquid |
| --- | --- | --- |
| Thawed probiotic bacteria | 0 | 79.20 |
| Sweet Whey | 16.81 | 0 |
| Dipotassium phosphate | 1.74 | 0 |
| Ground Flax seed | 0.84 | 0 |
| Flax oil | 0 | 1.41 |

Formulation 30

| MME ingredient | % solids | % liquid |
| --- | --- | --- |
| Thawed probiotic bacteria | 0 | 77.95 |
| Sucrose | 19.66 | 0 |
| Dipotassium phosphate | 0.64 | 0 |
| Flax oil | 0 | 1.75 |

Formulation 31

| MME ingredient | % solids | % liquid |
| --- | --- | --- |
| Thawed probiotic bacteria | 0 | 89.98 |
| Hemp seed powder | 6.64 | 0 |
| Ground Flax seed | 0.91 | 0 |
| Dipotassium phosphate | 0.72 | 0 |
| Flax oil | 0 | 1.75 |

Formulation 32

| MME ingredient | % solids | % liquid |
| --- | --- | --- |
| Thawed probiotic bacteria | 0 | 77.33 |
| Sucrose | 19.52 | 0 |
| Ground Flax seed | 0.78 | 0 |
| Dipotassium phosphate | 0.62 | 0 |
| Flax oil | 0 | 1.75 |

Formulation 33

| MME ingredient | % solids | % liquid |
| --- | --- | --- |
| Thawed probiotic bacteria | 0 | 73.61 |
| Sucrose | 18.60 | 0 |
| Hemp seed powder | 5.45 | 0 |
| Dipotassium phosphate | 0.59 | 0 |
| Flax oil | 0 | 1.75 |

Formulation 34

| MME ingredient | % solids | % liquid |
| --- | --- | --- |
| Thawed probiotic bacteria | 0 | 73.02 |
| Sucrose | 18.45 | 0 |

(continued)

| MME ingredient | % solids | % liquid |
| --- | --- | --- |
| Hemp seed powder | 5.45 | 0 |
| Dipotassium phosphate | 0.59 | 0 |
| Ground Flax seed | 0.74 | 0 |
| Flax oil | 0 | 1.75 |

Formulation 35

| MME ingredient | % solids | % liquid |
| --- | --- | --- |
| Thawed probiotic bacteria | 0 | 73.02 |
| Sucrose | 18.45 | 0 |
| Hemp seed powder | 5.45 | 0 |
| Dipotassium phosphate | 0.59 | 0 |
| Oat flour | 0.74 | 0 |
| Flax oil | 0 | 1.75 |

Formulation 36

| MME ingredient | % solids | % liquid |
| --- | --- | --- |
| Thawed probiotic bacteria | 0 | 74.55 |
| Sucrose | 18.63 | 0 |
| Hemp seed powder | 5.47 | 0 |
| Oat flour | 0.75 | 0 |
| Dipotassium phosphate | 0.60 | 0 |

Formulation 37

| MME ingredient | % solids | % liquid |
| --- | --- | --- |
| Thawed probiotic bacteria | 0 | 75.00 |
| Sucrose | 18.75 | 0 |
| Hemp seed powder | 5.50 | 0 |
| Oat flour | 0.75 | 0 |

Formulation 38

| MME ingredient | % solids | % liquid |
| --- | --- | --- |
| Thawed probiotic bacteria | 0 | 74.55 |
| Trehalose | 18.63 | 0 |
| Hemp seed powder | 5.47 | 0 |
| Oat flour | 0.75 | 0 |
| Dipotassium phosphate | 0.60 | 0 |

Formulation 39

| MME ingredient | % solids | % liquid |
| --- | --- | --- |
| Thawed probiotic bacteria | 0 | 73.06 |
| Trehalose | 18.45 | 0 |

(continued)

| MME ingredient | % solids | % liquid |
|---|---|---|
| Hemp seed powder | 5.41 | 0 |
| Oat flour | 0.74 | 0 |
| Dipotassium phosphate | 0.59 | 0 |
| Flax oil | 0 | 1.75 |

Formulation 40

| MME ingredient | % solids | % liquid |
|---|---|---|
| Thawed probiotic bacteria | 0 | 73.69 |
| Trehalose | 22.62 | 0 |
| Hemp seed powder | 1.66 | 0 |
| Dipotassium phosphate | 0.72 | 0 |
| Flax oil | 0 | 1.31 |

Formulation 41

| MME ingredient | % solids | % liquid |
|---|---|---|
| Thawed probiotic bacteria | 0 | 73.69 |
| Trehalose | 21.02 | 0 |
| Hemp seed powder | 3.30 | 0 |
| Dipotassium phosphate | 0.68 | 0 |
| Flax oil | 0 | 1.31 |

Formulation 42

| MME ingredient | % solids | % liquid |
|---|---|---|
| Thawed probiotic bacteria | 0 | 73.69 |
| Trehalose | 18.87 | 0 |
| Hemp seed powder | 5.53 | 0 |
| Dipotassium phosphate | 0.60 | 0 |
| Flax oil | 0 | 1.31 |

Formulation 43

| MME ingredient | % solids | % liquid |
|---|---|---|
| Thawed probiotic bacteria | 0 | 73.69 |
| Trehalose | 15.41 | 0 |
| Hemp seed powder | 9.10 | 0 |
| Dipotassium phosphate | 0.49 | 0 |
| Flax oil | 0 | 1.31 |

Formulation 44

| MME ingredient | % solids | % liquid |
|---|---|---|
| Fresh, liquid cell concentrate of probiotic bacteria | 0 | 76.9 |
| SKP | 0 | 23.1 |

Formulation 45

| MME ingredient | % solids | % liquid |
|---|---|---|
| Fresh, liquid cell concentrate of probiotic bacteria | 0 | 75.00 |
| SKP | 0 | 22.38 |
| Canola oil | 0 | 2.62 |

Formulation 46

| MME ingredient | % solids | % liquid |
|---|---|---|
| Fresh, liquid cell concentrate of probiotic bacteria | 0 | 33.3 |
| SKP | 0 | 66.7 |

Formulation 47

| MME ingredient | % solids | % liquid |
|---|---|---|
| Fresh, liquid cell concentrate of probiotic bacteria | 0 | 32.5 |
| SKP | 0 | 65.0 |
| Canola oil | 0 | 2.5 |

Formulation 48

| MME ingredient | % solids | % liquid |
|---|---|---|
| Fresh, liquid cell concentrate of probiotic bacteria | 0 | 52.40 |
| Sucrose solution* | 0 | 13.10 |
| Potassium Phosphate solution** | 0 | 33.70 |

Formulation 49

| MME ingredient | % solids | % liquid |
|---|---|---|
| Fresh, liquid cell concentrate of probiotic bacteria | 0 | 51.03 |
| Sucrose solution* | 0 | 12.76 |
| Potassium Phosphate solution** | 0 | 33.70 |
| Canola oil | 0 | 2.51 |

*Sucrose: 60 g mixed in 100 g of water at ~140°F.
**Potassium phosphate solution: Mono-potassium phosphate (159 g) and dipotassium phosphate (203.6 g) mixed in 1637.4 g of water at ~140°F.

For each blended formulation, the pH was adjusted to give a final value of from about 6 to about 6.5 and the temperature was from about 24°C to about 30°C.

*Example 2*

Microencapsulation formulations comprising: (i) sucrose alone; (ii) sucrose and potassium buffer; (iii) sucrose, potassium buffer and a plant-based food oil were tested for their effects on stability of *B. lactis* Bi-07 after lyophilisation using high pressure drying at a pressure of 1000 mTorr and temperature of from 22 to 28°C. The results are shown in **Table 3.**

**Table 3**

| Formulation | Water activity ($a_W$) | % Recovery | T0 Cell counts after lyophilization (CFU/ g) | % Cell survival at 38°C | |
|---|---|---|---|---|---|
| | | | | 14 days | 30 days |
| Stability of microencapsulation formulations comprising food plant-based oils | | | | | |
| 1 | 0.128 | 48 | 2.52E+11 | 45.0 | 28.1 |
| 2 | 0.150 | 34 | 4.23E+11 | 47.0 | 32.9 |
| 4 (Canola oil) | 0.167 | 91 | 6.67E+11 | 72.4 | 58.2 |
| 5 (Flax oil) | 0.162 | 82 | 5.37E+11 | 70.4 | 53.9 |
| 6 (Olive oil) | 0.157 | 66 | 6.57E+11 | 69.2 | 57.8 |
| 7 (Soybean) oil | 0.143 | 89 | 6.83E+11 | 81.7 | 71.4 |
| 8 (Coconut oil) | 0.158 | 40 | 6.93E+11 | 70.8 | 53.0 |
| SKP control (low pressure drying) | 0.035 | 70 | 1.29E+12 | 2.8 | 0.0 |

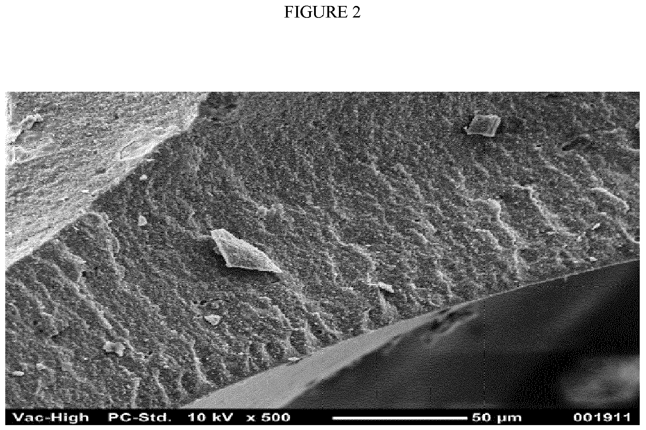

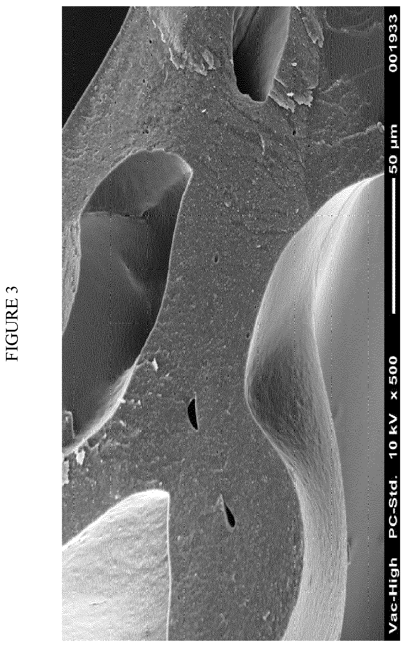

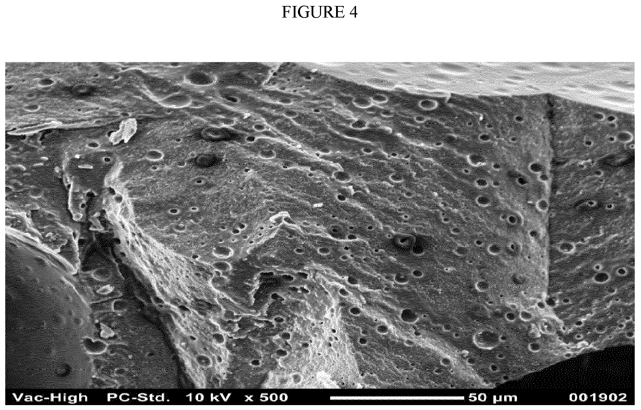

The SEM image of the lyophilised structure obtained for the SKP control, Formulation 2 and Formulation 4 are exemplified in **Figures 2 to 4,** respectively. Oil patches covering several tiers of pits can be seen in **Figure 4,** but are not present in **Figures 2 and 3.** These oil patches are believed to encapsulate and protect sheltered cells. Sharp ridges, such as those seen in **Figure 2,** suggest that cells became exposed to the environment. Fewer ridges and/or smooth structure suggest that fewer cells are exposed to environmental stresses such as air, water, oxygen and temperature *etc.* during freeze-drying.

The encapsulation ingredient sucrose alone (*i.e.,* Formulation 1) was observed to provide a cell survival of 28.1% at 38°C for 30 days. Further stability was observed when sucrose was mixed with small amounts of $K_2HPO_4$ (*i.e.,* Formulation 2), giving a cell survival of 32.9% at 38°C for 30 days. But an even greater improvement was observed when small amounts of food oil also was added (*i.e.,* Formulations 4-8). Specifically, addition of the tested plant-based food oils was observed to improve cell survival to approximately 53.0% to 71.4% at 38°C for 30 days.

### *Example 3*

Microencapsulation formulations comprising trehalose as the carbohydrate substrate were tested for their effects on stability of *B. lactis* Bi-07 after lyophilisation using high pressure drying at a pressure of 1000 mTorr and temperature of from 22 to 28°C. The results are shown in Table 4.

**Table 4**

| Formulation | Water activity ($a_W$) | T0 Cell counts after lyophilization (CFU/ g) | % Cell survival at 38°C | |
|---|---|---|---|---|
| | | | 14 days | 30 days |
| Stability of microencapsulation formulations comprising trehalose and plant-based food oils | | | | |
| 3 | 0.132 | 7.03E+11 | 77.0 | 43.7 |
| 9 (Canola oil) | 0.148 | 9.10E+11 | 84.8 | 42.1 |
| 10 (Flax oil) | 0.166 | 6.73E+11 | 102.6 | 50.7 |
| SKP control (low pressure drying) | 0.016 | 9.33E+11 | 10.6 | 4.0 |

### *Example 4*

Microencapsulation formulations comprising varying concentrations of canola, flax and soybean oil were tested for their effects on stability of *B. lactis* Bi-07 after lyophilisation using high pressure drying at a pressure of 1000 mTorr and temperature of from 22 to 28°C. The results are shown in Table 5.

**Table 5**

| Stability of microencapsulation formulations comprising varying amounts of plant-based food oil | | | | | | | | | |
|---|---|---|---|---|---|---|---|---|---|
| Formulation (% plant-based oil by weight) | Water activity (a$_W$) | T0 Cell counts after lyophilization (CFU/ g) | % Cell survival @ 38°C - Canola oil | | % Cell survival @ 38°C - Flax oil | | % Cell survival @ 38°C-Soybean oil | | |
| | | | 14 days | 30 days | 14 days | 30 days | 14 days | 30 days | |
| 2 (0%) | 0.150 | 4.23E+11 | 69.3 | 27.9 | 69.3 | 27.9 | 69.3 | 27.9 | |
| 12 (0.66%) | 0.201 | 4.51E+11 | 76.8 | 76.8 | 66.7 | 28.8 | 67.7 | 45 | |
| 13 (1.37%) | 0.171 | 4.50E+11 | 87.2 | 87.2 | 68.0 | 34.5 | 71.2 | 40.6 | |
| 14 (1.95%) | 0.179 | 6.52E+11 | 59.3 | 59.3 | 69.4 | 40.1 | 58.9 | 40.4 | |
| 15 (2.62%) | 0.192 | 4.74E+11 | 90.7 | 90.7 | 81.1 | 42.3 | 67.0 | 44.2 | |
| 16 (3.93%) | 0.175 | 9.10E+11 | 45.0 | 45.0 | 73.4 | 45.4 | 69.8 | 44.8 | |
| SKP control (low pressure drying) | 0.002 | 6.44E+11 | 45 | 17.5 | 45 | 17.5 | 45 | 17.5 | |

Plant-based food oil, particularly with inclusion rates of from 0.66% to 2.62% (by weight) in the final encapsulation formulation, was generally observed to provide improved stability of the probiotic despite high water activity. These formulations were also found to be stable under low pressure drying conditions (at a pressure of from about 50 to about 150 mTorr and temperature of from 2 to 32°C).

*Example 5*

Microencapsulation formulations comprising one or more fibre and/or protein rich substrates in conjunction with canola oil were tested for their effects on stability of *B. lactis* Bi-07 after lyophilisation using high pressure drying at a pressure of 1000 mTorr and temperature of from 22 to 28°C. The results are shown in Table 6.

**Table 6**

| Stability of microencapsulation formulations comprising canola oil and fibre and/or protein substrates | | | | |
|---|---|---|---|---|
| Formulation | Water activity (a$_W$) | T0 Cell counts after lyophilization (CFU/ g) | % Cell survival at at 38°C | |
| | | | 14 days | 30 days |
| 14 (Canola oil) | 0.188 | 6.13E+11 | 93.3 | 65.8 |
| 17 (Canola + brown rice protein) | 0.145 | 7.87E+11 | 95.59 | 55.25 |
| 18 (Canola + fenugreek seed flour) | 0.088 | 8.27E+11 | 68.47 | 39.52 |
| 19 (Canola + soybean flour & ground Flax seed) | 0.032 | 7.11E+11 | 50.5 | 24.2 |
| 20 (Canola + cocoa & ground flax seed) | 0.158 | 7.02E+11 | 67.9 | 37.0 |
| 21 (Canola + Zein protein & ground flax seed) | 0.127 | 7.45E+11 | 65.3 | 31.6 |
| 22 (Canola oil + brown rice, ground flax seed) | 0.149 | 7.87E+11 | 63.1 | 35.2 |
| 23 (Canola oil + whole egg protein, ground flax seed) | 0.115 | 7.17E+11 | 29.7 | 9.6 |
| 24 (Canola oil + egg white protein & ground flax seed) | 0.173 | 6.89E+11 | 57.7 | 20.5 |

(continued)

| Stability of microencapsulation formulations comprising canola oil and fibre and/or protein substrates | | | | |
|---|---|---|---|---|
| Formulation | Water activity (a_W) | T0 Cell counts after lyophilization (CFU/ g) | % Cell survival at at 38°C | |
| | | | 14 days | 30 days |
| 25 (Canola oil + ground fenugreek seed & ground flax seed) | 0.098 | 7.45E+11 | 61.7 | 27.9 |
| 26 (Canola oil + hemp seed powder & ground flax seed) | 0.245 | 5.83E+11 | 77.5 | 34.5 |

### Example 6

Microencapsulation formulations comprising sweet whey comprising lactose with canola oil were tested after low pressure drying (at a pressure of from about 50 to about 150 mTorr and temperature of from 2 to 32°C) for its effect on stability of *B. lactis* Bi-07 cultures. The results are shown in Table 7.

**Table 7**

| Stability of microencapsulation formulations comprising sweet whey powder | | | | |
|---|---|---|---|---|
| Formulation | Water activity (a_W) | T0 Cell counts after lyophilization (CFU/ g) | % Cell survival at @ 38°C | |
| | | | 14 days | 30 days |
| 28 | 0.046 | 5.26E+11 | 53.7 | 46.1 |
| 29 | 0.058 | 5.18E+11 | 76.9 | 59.9 |

### Example 7

Microencapsulation formulations comprising hemp were tested for their effects on stability of *B. lactis* Bi-07 after lyophilisation using high pressure drying at a pressure of 1000 mTorr and a pressure of from 22 to 28°C. The results are shown in Table 8.

**Table 8**

| Stability of microencapsulation formulations comprising Hemp seed powder | | | | |
|---|---|---|---|---|
| Formulation | Water activity (a_W) | T0 Cell counts after lyophilization (CFU/ g) | % Cell survival @ 38°C | |
| | | | 14 days | 30 days |
| 30 | 0.304 | 7.85E+11 | 80.5 | 67.1 |
| 31 | 0.033 | 1.01E+12 | 2.1 | 0.2 |
| 32 | 0.276 | 7.08E+11 | 79.5 | 72.1 |
| 33 | 0.295 | 7.22E+11 | 73.0 | 69.4 |
| 34 | 0.238 | 5.85E+11 | 80.9 | 77.2 |
| 35 | 0.242 | 6.39E+11 | 86.4 | 74.4 |
| SKP control (low pressure drying) | 0.047 | 5.93E+11 | 65.3 | 7.9 |

### Example 8

Microencapsulation formulations comprising canola oil were tested for their effects on stability of fresh liquid culture *B. lactis* Bi-07 after lyophilisation using low pressure drying (at a pressure of from about 50 to about 150 mTorr and temperature of from 2 to 32°C) using Formulations 44-49. The results are shown in Table 9.

**Table 9**

| Stability of microencapsulation formulations using fresh liquid cell concentrate of bacteria with low pressure drying* | | | |
|---|---|---|---|
| MME Formulation | Water activity ($a_W$) | % Cell survival (14 days) | % Cell survival (30 days) |
| 44. SKP (30%) | 0.001 | 73.2 | 50.6 |
| 45. SKP (30%) + 2.62% canola oil | 0.004 | 66.2 | 46.3 |
| 46. SKP (200%) | 0.160 | 20.0 | 12.3 |
| 47. SKP (200%), 2.5% canola oil | 0.187 | 27.5 | 21.3 |
| 48. Sucrose, potassium phosphate | 0.007 | 62.0 | 36.7 |
| 49. Sucrose, potassium phosphate, 2.51% canola oil | 0.006 | 66.9 | 46.5 |

*Example 9*

Microencapsulation formulations comprising small amounts of banana powder ("BP") were tested for their effects on stability of *B. lactis* Bi-07 after lyophilisation using low pressure drying (at a pressure of from about 50 to about 150 mTorr and temperature of from 2 to 32°C). The formulations tested are described in Table 10 (composition weights are given in grams).

**Table 10**

| Formulations comprising banana powder | | | | | | | | | |
|---|---|---|---|---|---|---|---|---|---|
| Formulation | Thawed Bi-07 (g) | Flax Oil (g) | Trehalose (g) | BP (g) | Hemp seed powder (g) | Oat Flour (g) | Ground Flax seed (g) | $K_2HPO_4$ ( g) | % solids |
| Control | 154 | 2.70 | 37.50 | 0 | 11.0 | 1.50 | 0 | 1.20 | 25 |
| BP (0.48%) | 157 | 2.75 | 37.50 | 1.0 | 11.0 | 1.50 | 0 | 1.20 | 25 |
| BP (1.38%) | 163 | 2.85 | 37.50 | 3.0 | 11.0 | 1.50 | 0 | 1.20 | 25 |
| BP (1.42%) | 158 | 2.77 | 37.50 | 3.0 | 11.0 | 0 | 0 | 1.20 | 25 |
| BP (1.73%) | 130 | 2.28 | 37.50 | 3.0 | 0 | 0 | 1.50 | 1.20 | 25 |
| BP (2.62%) | 172 | 3.02 | 37.50 | 6.0 | 11.0 | 1.50 | 0 | 1.20 | 25 |
| BP (3.73%) | 181 | 3.17 | 37.50 | 9.0 | 11.0 | 1.50 | 0 | 1.20 | 25 |

The results are shown in Table 11 (with the banana powder percentages being by weight).

**Table 11**

| Stability of microencapsulation formulations comprising banana powder | | | | | |
|---|---|---|---|---|---|
| Formulation | Water activity ($a_W$) | T0 Cell counts after lyophilization (CFU/ g) | % Cell survival at @ 38°C | | |
| | | | 14 days | 30 days | 60 days |
| Control | 0.014 | 2.71E+11 | 79 | 45 | 17 |
| BP (0.48%) | 0.012 | 2.49E+11 | >100 | 45 | 22 |
| BP (1.38%) | 0.011 | 2.43E+11 | >100 | 62 | 36 |
| BP (1.42%) | 0.013 | 2.19E+11 | >100 | 64 | 28 |
| BP (1.73%) | 0.027 | 2.42E+11 | >100 | >100 | 50 |

(continued)

| Stability of microencapsulation formulations comprising banana powder | | | | | |
|---|---|---|---|---|---|
| Formulation | Water activity ($a_W$) | T0 Cell counts after lyophilization (CFU/ g) | % Cell survival at @ 38°C | | |
| | | | 14 days | 30 days | 60 days |
| BP (2.62%) | 0.019 | 2.34E+11 | >100 | 55 | 28 |
| BP (3.73%) | 0.018 | 2.84E+11 | 90 | 46 | 27 |

Small amounts of banana powder as a secondary carbohydrate substrate were observed to give a benefit in cell survival (and thus stability), particularly at concentrations of up to 2.62% by weight (and even more particularly at concentrations of up to 1.73% by weight) and over time frames of up to at least 60 days.

*Example 10*

Bi-07 cell stability under low pressure drying was assessed for the various formulations shown in **Table 12** containing thawed frozen pellet cell concentrate of Bi-07. The low-pressure drying was conducted at a pressure of 50 to 150 mTorr and a temperature of from 2 to 32°C. The results are shown in **Table 13.**

**Table 12**

| Various formulations containing thawed frozen pellet Bi-07 cell concentrate | | | | | |
|---|---|---|---|---|---|
| Formulation | Thawed Bi-07 (%) | $K_2HPO_4$ (%) | Canola Oil (%) | Sucrose (%) | SKP (%) |
| Bi-07 alone | 100 | 0 | 0 | 0 | 0 |
| $K_2HPO_4$ + Bi-07 | 98.88 | 1.12 | 0 | 0 | 0 |
| Canola oil + Bi-07 | 98.04 | 0 | 1.96 | 0 | 0 |
| Sucrose + Bi-07 | 77.97 | 0 | 0 | 22.03 | 0 |
| $K_2HPO_4$ + canola oil + Bi-07 | 96.96 | 1.09 | 1.95 | 0 | 0 |
| Sucrose + $K_2HPO_4$ + Bi-07 | 77.08 | 1.12 | 0 | 21.80 | 0 |
| Sucrose + canola oil + Bi-07 | 76.41 | 0 | 1.99 | 21.60 | 0 |
| Sucrose + $K_2HPO_4$ + canola oil + Bi-07 | 75.57 | 1.10 | 1.96 | 21.37 | 0 |
| SKP control | 76.92 | 0 | 0 | 0 | 23.08 |

**Table 13**

| Cell survival for formulations of Table 12 subjected to low pressure drying | | |
|---|---|---|
| Formulation | $a_w$ | % Cell survival (30 days at 38°C) |
| Thawed frozen pellet Bi-07 cell concentrate | 0.033± 0.0037 | 0.0± 0.0 |
| $K_2HPO_4$ + Thawed frozen pellet Bi-07 cell concentrate | 0.033± 0.0045 | 0± 0 |
| Formulation | $a_w$ | % Cell survival (30 days at 38°C) |
| Canola oil + Thawed frozen pellet Bi-07 cell concentrate | 0.043± 0.0265 | 0.01± 0.01 |
| Sucrose + Thawed frozen pellet Bi-07 cell concentrate | 0.086± 0.0174 | 59± 8.14 |
| $K_2HPO_4$ + canola oil + Thawed frozen pellet Bi-07 cell concentrate | 0.040± 0.0015 | 0.11± 0.05 |

(continued)

| Formulation | $a_w$ | % Cell survival (30 days at 38°C) |
|---|---|---|
| Sucrose + $K_2HPO_4$ + Thawed frozen pellet Bi-07 cell concentrate | $0.050 \pm 0.0010$ | $48.8 \pm 7.2$ |
| Sucrose + canola oil + Thawed frozen pellet Bi-07 cell concentrate | $0.034 \pm 0.0037$ | $45.3 \pm 9.96$ |
| Sucrose + $K_2HPO_4$ + canola oil + Thawed frozen pellet Bi-07 cell concentrate | $0.044 \pm 0.0120$ | $62.2 \pm 4.4$ |
| SKP control (low pressure drying) | $0.042 \pm 0.0070$ | $41.5 \pm 1.65$ |

*Example 11*

Bi-07 cell stability under low pressure drying was assessed for the various formulations shown in **Table 14** containing fresh liquid cell concentrate of Bi-07. The low-pressure drying was conducted at a pressure of 50 to 150 mTorr and a temperature of from 2 to 32°C. The results are shown in **Table 15.**

**Table 14**

| Various formulations containing fresh liquid Bi-07 cell concentrate | | | | | |
|---|---|---|---|---|---|
| Formulations | Fresh, liquid cell concentrate Bi-07 (%) | $K_2HPO_4$ (%) | Canola Oil (%) | Sucrose (%) | SKP (%) |
| Bi-07 alone | 100 | 0 | 0 | 0 | 0 |
| $K_2HPO_4$ + Bi-07 | 98.88 | 1.12 | 0 | 0 | 0 |
| Canola oil + Bi-07 | 98.04 | 0 | 1.96 | 0 | 0 |
| Sucrose + Bi-07 | 77.97 | 0 | 0 | 22.03 | 0 |
| $K_2HPO_4$ + canola oil + Bi-07 | 96.96 | 1.09 | 1.95 | 0 | 0 |
| Sucrose + $K_2HPO_4$ + Bi-07 | 77.08 | 1.12 | 0 | 21.80 | 0 |
| Sucrose + canola oil + Bi-07 | 76.41 | 0 | 1.99 | 21.60 | 0 |
| Sucrose + $K_2HPO_4$ + canola oil + Bi-07 | 75.57 | 1.10 | 1.96 | 21.37 | 0 |
| SKP control | 76.92 | 0 | 0 | 0 | 23.08 |

**Table 15**

| Cell survival for formulations of Table 14 subjected to low pressure drying | | |
|---|---|---|
| Formulation | $a_w$ | % Cell survival (30 days at 38°C) |
| Bi-07 liquid cell concentrate | $0.033 \pm 0.0037$ | $0.1 \pm 0.0$ |
| $K_2HPO_4$ + Bi-07 liquid cell concentrate | $0.034 \pm 0.0045$ | $0.1 \pm 0.11$ |
| Canola oil + Bi-07 liquid cell concentrate | $0.034 \pm 0.0265$ | $0.1 \pm 0$ |
| Sucrose + Bi-07 liquid cell concentrate | $0.035 \pm 0.0174$ | $61.8 \pm 5.97$ |
| $K_2HPO_4$ + canola oil + Bi-07 liquid cell concentrate | $0.032 \pm 0.0015$ | $0.2 \pm 0.11$ |
| Sucrose + $K_2HPO_4$ + Bi-07 liquid cell concentrate | $0.047 \pm 0.0010$ | $77.7 \pm 11.30$ |
| Sucrose+ canola oil + Bi-07 liquid cell concentrate | $0.048 \pm 0.0037$ | $41.5 \pm 3.72$ |

(continued)

| Formulation | $a_w$ | % Cell survival (30 days at 38°C) |
|---|---|---|
| Sucrose+ $K_2HPO_4$ + canola oil + Bi-07 liquid cell concentrate | $0.029\pm 0.0120$ | $60.8\pm 3.40$ |
| SKP Control (low pressure drying) | $0.028\pm 0.0070$ | $93.4\pm 2.85$ |

The words "comprise", "comprises" and "comprising" are to be interpreted inclusively rather than exclusively. This interpretation is intended to be the same as the interpretation that these words are given under United States patent law at the time of this filing.

The singular forms "a" and "an" are intended to include plural referents unless the context dictates otherwise. Thus, for example, a reference to the presence of "an excipient" does not exclude the presence of multiple excipients unless the context dictates otherwise.

REFERENCES

All references cited in this specification are incorporated by reference into this specification.

Lamendella R, Santo Domingo JW, Kelty C, Oerther DB, "Bifidobacteria in feces and environmental waters," App. Environ. Microbiol., vol. 74, pp. 575-584 (2008).

Schell MA, Karmirantzou M, Snel B, Vilanova D, Berger B, Pessi G, Zwahlen MWC, Desiere F, Bork P, Delley M, Pridmore RD, Arigoni F., "The genome sequence of Bifidobacterium-longum reflects its adaptation to the human gastrointestinal tract." Proc. Natl. Acad. Sci. USA, vol. 99, pp. 14422-14427 (2002).

Scardovi V., "Genus Bifidobacterium," pp. 1418-1434 in Sheath PHA, Maiz NS, Sharp ME, Holt JG(ed), Bergey manual of systematic bacteriology, vol. 2, Williams & Wilkins, Baltimore (1986).

Stahl B, Barrangou R, "Complete Genome Sequences of Probiotic Strains Bifidobacterium animalis subsp. lactis B420 and Bi-07" J. Bacteriol. vol. 194(15) pp. 4131-4132 (2012).

Turroni F, Foroni E, Pizzetti P, Giubellini V, Ribbera A, Mausi P, Cangas's P, Bizzarri B, de Angelis GL, Shanahan F, van Sincere D, Ventura M., "Exploring the diversity of the Bifidobacterial population in the human intestinal tract," App. Environ. Microbial., vol. 75, pp. 1534-1545 (2009).

Turroni F, Marches' JR, Foroni E, Gabionade M, Shanahan F, Margolles A, van sundered D, Ventura M., "Macrobiotic analysis of the Bifidobacterial population in the human distal gut," ISME J., vol. 3, pp. 745-51 (2009).

Ventura M, Cachaça C, Tauti A, Chandra G, Fitzgerald GF, Chater KF, Van Sinderen D., "Genomics of Actinobacteria: tracing the evolutionary history of an ancient phylum," Microbial. Mol. Biol. Rev., vol. 71, pp. 495-548 (2007).

Ventura M, Margolles A, Turroni F, Zomer A, de los ReyesWGailan CG, van Sinderen D., "Stress responses of Bifidobacteria," pp 323c438 in Tsakani E, Papadimitriou K (ed), Stress response of lactic acid bacteria (Food microbiology and food safety), Springer Science Business Media Inc., New York, NY (2011).

Yang YWX, He M, Hu G, Wei J, Pages P, Yang XWH, Board W Naturel S., "Effect of a fermented milk containing Bifidobacterium-lactis DNc173010 on Chinese constipated women," World J. Gastroenterol., vol. 14, pp. 6237-43 (2008).

**Claims**

1. A composition for stabilizing probiotic bacteria, wherein the composition comprises:

    i) a first carbohydrate substrate,
    ii) a potassium buffer salt, and
    iii) a plant-based food oil.

2. A composition according to claim 1, wherein the first carbohydrate substrate comprises a substrate selected from lactose, trehalose and sucrose.

3. A composition according to any one of the preceding claims, wherein the plant-based food oil comprises an oil selected from soybean oil, coconut oil, olive oil, sunflower oil, flax oil and canola oil.

**4.** A composition according to any one of the preceding claims, wherein the potassium buffer salt comprises dipotassium phosphate.

**5.** A composition according to any one of the preceding claims, wherein the composition further comprises whey, soybean protein, cocoa, brown rice protein, ground fenugreek seed, whole egg protein, egg white protein, Zain protein, hemp seed powder, brown rice protein, oat flour, flax seed flour or ground flax seed.

**6.** A composition according to any one of the preceding claims, wherein the composition further comprises banana powder.

**7.** A matricial microencapsulation (MME) formulation, wherein the formulation comprises:

a probiotic bacterial culture, and
a composition according to any one of the preceding claims.

**8.** A formulation according to claim 8, wherein:

the first carbohydrate substrate is present in the formulation at a concentration of from 10% to 30% by weight,
the potassium buffer salt is present in the formulation at a concentration of no greater than 2% by weight, and
the plant-based food oil is present in the formulation at a concentration of no greater than 4.0% by weight.

**9.** A formulation according to claim 7 or 8, wherein the probiotic bacterial culture comprises *Bifidobacteria.*

**10.** A process for preserving a probiotic bacterial culture, wherein:

the process comprises admixing the probiotic bacterial culture with a composition according to any one of claims 1 to 6 to form a formulation in accordance with any one of claims 7 to 9; and
subjecting the formulation to lyophilization to form a lyophilized probiotic composition.

**11.** A process according to claim 10, wherein the lyophilization comprises a drying step conducted at a pressure of from 50 to 150 mTorr and a temperature of from 2 to 32°C.

**12.** A process according claims 10, wherein the lyophilization comprises a drying step conducted at a pressure of 900 to 1100 mTorr and a temperature of from 22 to 28°C.

**13.** A process according to any one of claims 10 to 12, wherein:

the probiotic bacterial culture is in the form of a frozen pellet before the admixing, and
the frozen pellet is melted before the admixing.

**14.** A lyophilized probiotic composition, wherein:

the composition is produced by the process according to any one of claims 10 to 13, and
the composition can be characterized as exhibiting a cell survival of at least 50% when stored for 30 days at a temperature of 38°C.

**15.** A lyophilized probiotic composition, wherein:

the composition is produced by the process according to any one of claims 10 to 13, and
the composition can be characterized as exhibiting a cell survival of at least 40% when stored for 60 days at a temperature of 38°C.

FIGURE 1

FIGURE 2

FIGURE 3

FIGURE 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201666<br>Thomson Scientific, London, GB;<br>AN 2016-352965<br>XP002781375,<br>-& CN 105 567 669 A (WEI Y)<br>11 May 2016 (2016-05-11)<br>* abstract *<br>* examples 1-3 *<br>* claims 1-10 *<br>----- | 1-15 | INV.<br>A01N1/02<br>A23L3/00<br>A01N63/00<br>A01N63/02<br>A61K35/745<br>C12N1/00<br>A23L33/125<br>A01N25/28<br>A01P1/00 |
| X | DATABASE WPI<br>Week 201632<br>Thomson Scientific, London, GB;<br>AN 2015-803018<br>XP002781376,<br>-& CN 105 053 251 A (TORADOR DAIRY IND TIANJIN CO LTD)<br>18 November 2015 (2015-11-18)<br>* abstract *<br>* claims 1-10 *<br>* examples 1-5 *<br>----- | 1-15 | |
| X | WO 2011/004375 A1 (RUBIN ISRAEL [IL]; ZIMAND HENRI [MC]; SASON DORON [IL]; PENHASI ADEL [])<br>13 January 2011 (2011-01-13)<br>* claims 1-15 *<br>* examples 1-13 *<br>----- | 1-15 | **TECHNICAL FIELDS SEARCHED (IPC)**<br>A01N<br>A23L<br>A61K<br>C12N |
| X | US 2010/303962 A1 (PENHASI ADEL [IL] ET AL) 2 December 2010 (2010-12-02)<br>* claims 1-17 *<br>* examples 1-3 *<br>-----<br>-/-- | 1-15 | |

The present search report has been drawn up for all claims

| Place of search | Date of completion of the search | Examiner |
|---|---|---|
| Munich | 25 May 2018 | Marie, Gérald |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 4

Europäisches Patentamt
European Patent Office
Office européen des brevets

**EUROPEAN SEARCH REPORT**

Application Number

EP 18 16 1269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 501 258 B1 (FEREGRINO-QUEZADA JOSE ANTONIO [MX] ET AL) 6 August 2013 (2013-08-06) * claims 1-13 * * table 2 * | 1-15 | |
| X | WO 2011/031149 A1 (MEAD JOHNSON NUTRITION CO [US]; VAN TOL ERIC A F [NL]; RUSSEL WILLIAM) 17 March 2011 (2011-03-17) * claims 1-13 * * page 8, last paragraph - page 10, paragraph 1; examples 1-5 * * page 13, last paragraph - page 14, paragraph 1 * * page 12, paragraph 2 * | 1-15 | |
| X | DATABASE WPI Week 201817 Thomson Scientific, London, GB; AN 2018-12196G XP002781377, -& CN 107 647 090 A (WUHAN XINWANG FEED TECHNOLOGY CO LTD) 2 February 2018 (2018-02-02) * abstract * * claims 1-10 * * paragraphs [0040] - [0043], [0074] - [0075], [0085] * | 1-15 | TECHNICAL FIELDS SEARCHED (IPC) |
| X | US 2016/015068 A1 (AO ZIHUA [US] ET AL) 21 January 2016 (2016-01-21) * claims 1-20 * * tables 1-6 * * paragraphs [0095], [0111], [0131], [0132], [0133] * * examples 1-4 * | 1-15 | |

-/--

The present search report has been drawn up for all claims

| Place of search | Date of completion of the search | Examiner |
|---|---|---|
| Munich | 25 May 2018 | Marie, Gérald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 18 16 1269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/282304 A1 (CHUNG MYUNG JUN [KR]) 8 November 2012 (2012-11-08) * claims 1-17 * * examples 1-4 * | 1-15 | |
| X | DATABASE WPI Week 201539 Thomson Scientific, London, GB; AN 2015-342709 XP002781378, -& CN 104 489 101 A (AUSNUTRIA DAIRY CHINA CO LTD) 8 April 2015 (2015-04-08) * abstract; claims 1-5 * * examples 1,2 * | 1-15 | |
| X | DATABASE WPI Week 199850 Thomson Scientific, London, GB; AN 1998-592555 XP002781379, -& RU 2 108 724 C1 (CHILD FEEDSTUFFS RES INST) 20 April 1998 (1998-04-20) * abstract * * claims 1-2 * * examples 1-5 * * paragraphs [0011], [0036], [0063] * | 1-15 | TECHNICAL FIELDS SEARCHED (IPC) |
| X | DATABASE WPI Week 201629 Thomson Scientific, London, GB; AN 2015-80301W XP002781380, -& CN 105 053 217 A (HYPROCA NOURISHMENT CO LTD) 18 November 2015 (2015-11-18) * abstract * * claims 1-7 * * example 1 * | 1-15 | |

-/--

The present search report has been drawn up for all claims

| Place of search | Date of completion of the search | Examiner |
|---|---|---|
| Munich | 25 May 2018 | Marie, Gérald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches
Patentamt

European
Patent Office

Office européen
des brevets

# EUROPEAN SEARCH REPORT

Application Number

EP 18 16 1269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/042932 A1 (SOLAE LLC [US]; MALDONADO YADILKA [US]; SMITH WILLIAM C [US]; NGUYEN M) 15 April 2010 (2010-04-15) * claims 1-18 * * examples 1-2 * | 1-15 | |
| X | GB 1 271 674 A (NISSHIN FLOUR MILLING CO [JP]) 26 April 1972 (1972-04-26) * example 4 * | 1-15 | |
| X | WO 2014/082132 A1 (PROGEL PTY LTD [AU]) 5 June 2014 (2014-06-05) * claims 1-17 * * examples 1-23 * * page 50, paragraph 1 * * page 34, paragraph 3 - page 35, column 1 * | 1-15 | |
| X | US 2013/089638 A1 (AO ZIHUA [US] ET AL) 11 April 2013 (2013-04-11) * claims 1-20 * * paragraphs [0018], [0020], [0022], [0032], [0033] * | 1-15 | TECHNICAL FIELDS SEARCHED (IPC) |
| X | WO 2005/030229 A1 (COMMW SCIENT IND RES ORG [AU]; CRITTENDEN ROSS [AU]; SANGUANSRI LUZ [A] 7 April 2005 (2005-04-07) * examples 1-12 * * page 19, paragraph 3 - page 21, paragraph 1 * | 1-15 | |
| X | US 2017/296598 A1 (MADHAVAMENON KRISHNAKUMAR ILLATHU [IN] ET AL) 19 October 2017 (2017-10-19) * examples 1-5 * * claims 1-16 * | 1-15 | |

The present search report has been drawn up for all claims

| Place of search | Date of completion of the search | Examiner |
|---|---|---|
| Munich | 25 May 2018 | Marie, Gérald |

EPO FORM 1503 03.82 (P04C01)

## ANNEX TO THE EUROPEAN SEARCH REPORT
## ON EUROPEAN PATENT APPLICATION NO.

EP 18 16 1269

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2018

| Patent document cited in search report | | Publication date | Patent family member(s) | | Publication date |
|---|---|---|---|---|---|
| CN 105567669 | A | 11-05-2016 | NONE | | |
| CN 105053251 | A | 18-11-2015 | NONE | | |
| WO 2011004375 | A1 | 13-01-2011 | AU | 2010202518 A1 | 08-07-2010 |
| | | | AU | 2010269814 A1 | 01-03-2012 |
| | | | CA | 2767602 A1 | 13-01-2011 |
| | | | CN | 102595938 A | 18-07-2012 |
| | | | CN | 105105104 A | 02-12-2015 |
| | | | EP | 2451300 A1 | 16-05-2012 |
| | | | NZ | 597992 A | 29-11-2013 |
| | | | RU | 2012104796 A | 20-08-2013 |
| | | | US | 2010303962 A1 | 02-12-2010 |
| | | | US | 2011008493 A1 | 13-01-2011 |
| | | | US | 2013115334 A1 | 09-05-2013 |
| | | | WO | 2011004375 A1 | 13-01-2011 |
| US 2010303962 | A1 | 02-12-2010 | AU | 2010202518 A1 | 08-07-2010 |
| | | | AU | 2010269814 A1 | 01-03-2012 |
| | | | CA | 2767602 A1 | 13-01-2011 |
| | | | CN | 102595938 A | 18-07-2012 |
| | | | CN | 105105104 A | 02-12-2015 |
| | | | EP | 2451300 A1 | 16-05-2012 |
| | | | NZ | 597992 A | 29-11-2013 |
| | | | RU | 2012104796 A | 20-08-2013 |
| | | | US | 2010303962 A1 | 02-12-2010 |
| | | | US | 2011008493 A1 | 13-01-2011 |
| | | | US | 2013115334 A1 | 09-05-2013 |
| | | | WO | 2011004375 A1 | 13-01-2011 |
| US 8501258 | B1 | 06-08-2013 | NONE | | |
| WO 2011031149 | A1 | 17-03-2011 | AU | 2010293145 A1 | 08-03-2012 |
| | | | BR | 112012005533 A2 | 26-04-2016 |
| | | | CA | 2772593 A1 | 17-03-2011 |
| | | | CN | 102575224 A | 11-07-2012 |
| | | | EC | SP12011786 A | 31-07-2012 |
| | | | EP | 2295535 A1 | 16-03-2011 |
| | | | EP | 2475764 A1 | 18-07-2012 |
| | | | ES | 2614929 T3 | 02-06-2017 |
| | | | JP | 2013504321 A | 07-02-2013 |
| | | | KR | 20120061836 A | 13-06-2012 |
| | | | MX | 345077 B | 16-01-2017 |
| | | | MY | 160646 A | 15-03-2017 |
| | | | NZ | 597818 A | 31-01-2014 |
| | | | PE | 15572012 A1 | 21-11-2012 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                EP 18 16 1269

This annex lists the patent family members relating to the patent documents cited in  the above-mentioned European search report.
The members are as contained in the European Patent Office EDP file on
The European Patent Office is in no way liable for these particulars which are merely given for the purpose of information.

25-05-2018

| Patent document cited in search report | Publication date | Patent family member(s) | Publication date |
|---|---|---|---|
| | | PL 2475764 T3 | 30-06-2017 |
| | | RU 2012114190 A | 20-10-2013 |
| | | SG 178230 A1 | 29-03-2012 |
| | | TW 201124529 A | 16-07-2011 |
| | | US 2011217402 A1 | 08-09-2011 |
| | | WO 2011031149 A1 | 17-03-2011 |
| CN 107647090 A | 02-02-2018 | NONE | |
| US 2016015068 A1 | 21-01-2016 | AR 101192 A1 | 30-11-2016 |
| | | AU 2015290178 A1 | 12-01-2017 |
| | | CA 2955158 A1 | 21-01-2016 |
| | | CN 107072279 A | 18-08-2017 |
| | | EP 3169167 A1 | 24-05-2017 |
| | | PH 12017500074 A1 | 15-05-2017 |
| | | SG 11201610426Q A | 27-01-2017 |
| | | TW 201613488 A | 16-04-2016 |
| | | US 2016015068 A1 | 21-01-2016 |
| | | WO 2016010664 A1 | 21-01-2016 |
| US 2012282304 A1 | 08-11-2012 | EP 2653533 A2 | 23-10-2013 |
| | | KR 20120046676 A | 10-05-2012 |
| | | US 2012282304 A1 | 08-11-2012 |
| | | WO 2012060554 A2 | 10-05-2012 |
| CN 104489101 A | 08-04-2015 | NONE | |
| RU 2108724 C1 | 20-04-1998 | NONE | |
| CN 105053217 A | 18-11-2015 | NONE | |
| WO 2010042932 A1 | 15-04-2010 | BR PI0914023 A2 | 26-07-2016 |
| | | CA 2735153 A1 | 15-04-2010 |
| | | CN 102176835 A | 07-09-2011 |
| | | EP 2330920 A1 | 15-06-2011 |
| | | JP 5619014 B2 | 05-11-2014 |
| | | JP 2012504971 A | 01-03-2012 |
| | | KR 20110084909 A | 26-07-2011 |
| | | US 2011183900 A1 | 28-07-2011 |
| | | WO 2010042932 A1 | 15-04-2010 |
| GB 1271674 A | 26-04-1972 | DE 1934652 A1 | 22-01-1970 |
| | | FR 2014544 A1 | 17-04-1970 |
| | | GB 1271674 A | 26-04-1972 |
| WO 2014082132 A1 | 05-06-2014 | AU 2013351920 A1 | 02-07-2015 |

EPO FORM P0459

For more details about this annex : see Official Journal of the European Patent Office, No. 12/82

**ANNEX TO THE EUROPEAN SEARCH REPORT**

**ON EUROPEAN PATENT APPLICATION NO.**                    EP 18 16 1269

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2018

| Patent document cited in search report | Publication date | Patent family member(s) | Publication date |
|---|---|---|---|
| | | CA       2931835 A1 | 05-06-2014 |
| | | DK       2925300 T3 | 26-02-2018 |
| | | EP       2925300 A1 | 07-10-2015 |
| | | JP    2016501868 A | 21-01-2016 |
| | | KR   20160018448 A | 17-02-2016 |
| | | US    2015313844 A1 | 05-11-2015 |
| | | WO    2014082132 A1 | 05-06-2014 |
| US 2013089638    A1 | 11-04-2013 | AR       088035 A1 | 07-05-2014 |
| | | BR 112014008498 A2 | 11-04-2017 |
| | | CA       2852109 A1 | 18-04-2013 |
| | | CN     103929979 A | 16-07-2014 |
| | | CN     107647394 A | 02-02-2018 |
| | | CO       6940418 A2 | 09-05-2014 |
| | | EP       2765872 A1 | 20-08-2014 |
| | | HK       1199797 A1 | 24-07-2015 |
| | | PE      20802014 A1 | 24-12-2014 |
| | | RU     2014110708 A | 20-11-2015 |
| | | SG 11201400696X A | 28-08-2014 |
| | | TW      201325460 A | 01-07-2013 |
| | | US    2013089638 A1 | 11-04-2013 |
| | | WO    2013055463 A1 | 18-04-2013 |
| WO 2005030229    A1 | 07-04-2005 | CA       2538676 A1 | 07-04-2005 |
| | | CN       1859920 A | 08-11-2006 |
| | | DK       1667696 T3 | 29-02-2016 |
| | | EP       1667696 A1 | 14-06-2006 |
| | | ES       2561831 T3 | 01-03-2016 |
| | | JP       6033998 B2 | 30-11-2016 |
| | | JP    2007507209 A | 29-03-2007 |
| | | JP    2010187670 A | 02-09-2010 |
| | | JP    2014140369 A | 07-08-2014 |
| | | JP    2016052323 A | 14-04-2016 |
| | | KR   20060092231 A | 22-08-2006 |
| | | KR   20120025005 A | 14-03-2012 |
| | | NZ        545925 A | 30-06-2008 |
| | | US    2007122397 A1 | 31-05-2007 |
| | | WO    2005030229 A1 | 07-04-2005 |
| US 2017296598    A1 | 19-10-2017 | NONE | |

EPO FORM P0459

For more details about this annex : see Official Journal of the European Patent Office, No. 12/82

# EP 3 524 051 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9055763 B

### Non-patent literature cited in the description

- **LAMENDELLA R ; SANTO DOMINGO JW ; KELTY C ; OERTHER DB.** Bifidobacteria in feces and environmental waters. *App. Environ. Microbiol.,* 2008, vol. 74, 575-584
- **SCHELL MA ; KARMIRANTZOU M ; SNEL B ; VILANOVA D ; BERGER B ; PESSI G ; ZWAHLEN MWC ; DESIERE F ; BORK P ; DELLEY M.** The genome sequence of Bifidobacterium-longum reflects its adaptation to the human gastrointestinal tract. *Proc. Natl. Acad. Sci. USA,* 2002, vol. 99, 14422-14427
- Genus Bifidobacterium. **SCARDOVI V.** Bergey manual of systematic bacteriology. Williams & Wilkins, 1986, vol. 2, 1418-1434
- **STAHL B ; BARRANGOU R.** Complete Genome Sequences of Probiotic Strains Bifidobacterium animalis subsp. lactis B420 and Bi-07. *J. Bacteriol.,* 2012, vol. 194 (15), 4131-4132
- **TURRONI F ; FORONI E ; PIZZETTI P ; GIUBELLINI V ; RIBBERA A ; MAUSI P ; CANGAS'S P ; BIZZARRI B ; DE ANGELIS GL ; SHANAHAN F.** Exploring the diversity of the Bifidobacterial population in the human intestinal tract. *App. Environ. Microbial.,* 2009, vol. 75, 1534-1545
- **TURRONI F ; MARCHES' JR ; FORONI E ; GABIONADE M ; SHANAHAN F ; MARGOLLES A ; VAN SUNDERED D ; VENTURA M.** Macrobiotic analysis of the Bifidobacterial population in the human distal gut. *ISME J.,* 2009, vol. 3, 745-51
- **VENTURA M ; CACHAÇA C ; TAUTI A ; CHANDRA G ; FITZGERALD GF ; CHATER KF ; VAN SINDEREN D.** Genomics of Actinobacteria: tracing the evolutionary history of an ancient phylum. *Microbial. Mol. Biol. Rev.,* 2007, vol. 71, 495-548
- Stress responses of Bifidobacteria. **VENTURA M ; MARGOLLES A ; TURRONI F ; ZOMER A ; DE LOS REYESWGAILAN CG ; VAN SINDEREN D.** Stress response of lactic acid bacteria (Food microbiology and food safety). Springer Science Business Media Inc, 2011, 323c438
- **YANG YWX ; HE M ; HU G ; WEI J ; PAGES P ; YANG XWH ; BOARD W NATUREL S.** Effect of a fermented milk containing Bifidobacterium-lactis DNc173010 on Chinese constipated women. *World J. Gastroenterol.,* 2008, vol. 14, 6237-43